# EUROPÄISCHE PATENTSCHRIFT

(11) **EP 2 080 483 B1**
(45) Veröffentlichungstag und Bekanntmachung des Hinweises auf die Patenterteilung: **05.06.2013**
(21) Anmeldenummer: 08171663.1
(22) Anmeldetag: 15.12.2008
(51) Int. Cl.: A61B 18/14

(54) **Ablationskatheter-Anordnung und Kühlungssteuerung**
Ablation catheter assembly and cooling control
Agencement de cathéter d'ablation et commande de refroidissement

(30) Priorität: 17.01.2008 DE 102008004972
(43) Veröffentlichungstag der Anmeldung: 22.07.2009
(73) Patentinhaber: Biotronik CRM Patent AG, 6341 Baar (CH)
(72) Erfinder: Jakus, Laszlo, 2095, Pilisszanto (HU)
(74) Vertreter: Lindner-Vogt, Karin L.

(56) Entgegenhaltungen:
- US-A- 5 893 884
- US-A- 6 053 912
- US-A1- 2003 004 506
- US-A1- 2005 090 814
- US-A1- 2005 171 582
- ANTOINE DA COSTA ET AL: "Catheter selection for ablation of the cavotricuspid isthmus for treatment of typical atrial flutter" JOURNAL OF INTERVENTIONAL CARDIAC ELECTROPHYSIOLOGY, KLUWER ACADEMIC PUBLISHERS, BO, Bd. 17, Nr. 2, 1. März 2007 (2007-03-01), Seiten 93-101, XP019480883 ISSN: 1572-8595

## Beschreibung

Die Erfindung betrifft eine Ablationskatheter-Anordnung zur hochfrequenten Verödung von Gewebe, insbesondere zum gezielten Erstellen von linearen und/oder punktuellen Läsionen in einem Herzgewebe.

Erkrankungen im Herzen sind weit verbreitet. Die häufigsten Krankheiten, die den Herzmuskel und nicht die Herzkranzgefäße betreffen, betreffen die Übertragung von Reizen. Bei einer normalen Erregungsleitung werden diese elektrischen Reize vom Sinusknoten, der sich im oder am rechten Herzohr befindet, gebildet und breiten sich über den AV-Knoten (Atrioventrikularknoten), das His-Bündel, die Tawara-Schenkel und die Purkinje-Fasern über den ganzen Herzmuskel aus, um so die Kontraktion des Herzen von der Herzspitze (Apex) aus über die Herzmuskulatur der beiden Ventrikel zu den Vorhöfen einzuleiten. Es entsteht dabei eine Kreisbahn.
Die störungsfreie Reiz- oder Erregungsleitung führt dann zum physiologisch störungsfreien Blutauswurf in die Lungenarterie und in die Aorta. Beachtenswert ist die Struktur des Herzgewebes, welches als ein Muskel verhält, der an allen Stellen in jede Richtung gleich erregbar ist. Bei einem gesunden Herzen wird nach einer erfolgen Aktivierung der Ventrikelmuskulatur die Erregungsleitung durch eine gleiche Polarisierung aller Muskelzellen geblockt, so dass keine Potentialdifferenz eine elektrische Reizleitung ermöglichen kann. Diesen Zustand nennt man Refraktärzeit, da alle Muskelzellen refraktär und nicht erregbar sind. Dieser Zustand wird von selbst nach kurzer Zeit aufgehoben, um so die nächste Erregung und somit den nächsten Herzschlag zu ermöglichen.

Liegt nun ein anatomisches oder funktionelles Hindernis vor - beispielsweise in einem Bündel einer sich aufzweigenden Purkinje-Faser, kann, bevor die Überleitung an dieser Stelle völlig blockiert ist, ein gefährliches Zwischenstadium eintreten: ein unidirektionaler Block. Dabei verlangsamt das geschädigte Areal den Durchtritt einer Erregung, bis es irgendwann gegenüber der folgenden Erregung refraktär wird, also schwer oder überhaupt nicht erregbar ist. Diese Erregung kann das geschädigte Areal nun aber in der Gegenrichtung durchlaufen, da sie dieses geschädigte Areal zu einem späteren Zeitpunkt erreicht, zu dem es möglicherweise nicht mehr refraktär ist. Die Laufzeitverzögerung entsteht dabei durch den "Umweg", den die Erregung laufen muss.
Ist nun die hinter dieser in entgegen gesetzter Richtung verlaufenden (Fachausdruck: retrograde Leitung) Erregung zurückbleibende Refraktärstrecke kürzer als die Kreisbahn, wird die Erregung innerhalb des Kreises nicht mehr gelöscht und kann ihn permanent durchlaufen. Man spricht von einer kreisenden Erregung.
Die Gefahr dabei besteht in einem Wiedereintritt ("reentry") der Erregungswelle in das umgebende Gewebe, wenn diese nicht mehr refraktär ist. Es kann sich eine Erregung ausbilden, die sich sozusagen selbst unterhält. Dies stellt die Ursache schwerer, mit der Gefahr des Flimmerns einhergehender tachykarder Rhythmusstörungen dar.

Neben den medikamentösen Therapien hat sich die elektrophysiologische Therapie durchgesetzt. Dabei werden gezielt bestimmte Stellen des Herzmuskels durch Hochfrequenz erhitzt und dadurch verödet oder ablatiert, um die elektrische Leitung zu verhindern. Verödetes Gewebe ist nicht mehr leitfähig.

Zur Ablationstherapie werden Ablationskatheter-Anordnungen eingesetzt.

Diese umfassen - wie der Name schon sagt - einen Ablationskatheter, und einen Ablationsgenerator und eine Kühlmittelpumpe.
Der Ablationskatheter ist ein lang gestreckter Katheter, welcher über ein Gefäß an die Behandlungsstelle geführt wird. Er umfasst eine elektrisch isolierende schlauchförmige Hülle und darin verlaufende elektrisch leitfähige Zuleitungen und Kühlmittelflussbahnen. Am distalen Ende befinden sich Elektrodenpole, die geeignet sind, hochfrequente Energieimpulse abzugeben. Die Elektrodenpole sind punkt- und/oder flächenförmig und sind mit den Zuleitungen elektrisch verbunden. Weiterhin kann ein Ablationskatheter ein Steuermittel enthalten, mit welchem der distale Katheterabschnitt beispielsweise in eine lineare oder kreisförmige Form verbringbar ist. Somit können lineare Läsionen oder kreisförmige Läsionen beispielsweise um die Mündung der Lungenvenen herum hergestellt werden.

Der Ablationsgenerator generiert ausgangsseitig ein hochfrequentes Energieimpulssignal oder eine hochfrequentes Energiefeld, welches an die Zuleitungen des Ablationskatheters weitergeleitet wird und von dort an die Elektrodenpole zur Abgabe an das Herzgewebe weitergeleitet werden. Der Generator wird von einem Operateur gesteuert. Ebenfalls vom Operateur wird eine Vorrichtung zur Kühlung von Ablationskathetern bedient, welche auf Anforderung des Operateurs ein flüssiges Kühlmedium (in der Regel eine physiologische Kochsalzlösung) in der mindestens einen Kühlmittelbahn des Ablationskatheters bereitstellt, wobei das Kühlmittel durch die Kühlmittelbahn bis zu den am distalen Abschnitt oder am distalen Ende befindlichen Elektrodenpolen führt. Dadurch wird sowohl der Katheter als auch die Ablationsstelle gekühlt, indem das Kühlmittel entweder wieder durch eine weitere Kühlmittelbahn durch den Katheter zurückgeführt wird und/oder durch Öffnungen im distalen Abschnitt an die Ablationsstelle geführt werden kann. Die Kühlvorrichtung weist weiterhin eine Steuerung auf, die die Pumpe so steuert, dass sie mindestens eine niedere und eine erhöhte Flussrate erzeugt. So sorgt die Kühlvorrichtung für einen gleichmäßigen immer währenden Kühlmittelfluss durch den Katheter zu den Elektrodenpolen mit einer niederen Flussrate. Veranlasst der Operater nun die Abgabe eines hochfrequenten Signals, so wird die erhöhte Abgabe von Kühlmittel gefordert. Diese Anforderung macht der Operateur, indem er gleichzeitig mit der Steuerung des Ablationsgenerators über ein Fußpedal bei der Kühlvorrichtung einen erhöhte Flussrate erzeugt. So wird eine noch stärkere Kühlung hervorgerufen.
Nachteilig an dieser manuellen Steuerung ist, dass der Operateur zwei unterschiedliche Systeme bedienen muss. Allein diese Bedienung erfordert so viel Konzentration und Übung, dass es leicht zu Fehlern in der Führung des Katheters kommen kann. Selbst wenn die Steuerung der Kühlvorrichtung durch einen Assistenten vorgenommen wird, erfordert die Koordination der Bedienung des Katheters, Ablationsgenerators und der Pumpe sehr viel Konzentration und Abstimmung.

Bekannte Weiterentwicklungen sind Ablationsanordnungen, die so aufgebaut sind, dass der Generator und eine spezifische, zum Generator kompatible Kühlvorrichtung zusammenwirken. Diese Anordnungen sind immer so aufeinander abgestimmt, dass der Austausch des Generators den gleichzeitigen Austausch der Kühlvorrichtung bedeutet. Der Generator schaltet bei Abgabe eines hochfrequenten Impulses die Flussrate der Pumpe auf einen erhöhten Kühlmittelfluss um. Sowohl der Impuls als auch das Kühlmittel werden über eine proprietäre Schnittstelle zwischen Katheter einerseits und Generator und Pumpe andererseits in den Katheter eingespeist. Nachteilig ist also, dass der Operateur auf genau ein solches Gerätepaar aus Ablationsgenerator und Kühlpumpe festgelegt ist.

Eine individuelle Anpassung, die sich auch manchmal aus der Indikation und dem damit verbundenen Kathetertyp oder der erforderlichen Energieabgabe ergibt, ist also nicht möglich. So kann es während einer Behandlung erforderlich sein, dass unter Beibehaltung der Kühlvorrichtung und der ständigen Kühlung mit dem niedrigen und erhöhten Kühlmittelfluss auf einen anderen Ablationsgenerator gewechselt werden muss, beispielsweise weil eine höhere Ablationsenergie erforderlich ist, ein nicht kompatibler Ablationskatheter verwendet werden muss oder aber weil der Ablationsgenerator schlicht defekt ist. Bei den bisher bekannten Ablationsanordnungen verliert der Anwender dabei die automatische Kopplung der Kühlvorrichtung an den Ablationsgenerator, da die Pumpe nur mit dem kompatiblen Generator zusammenwirkt. Die Kühlung des Katheters muss also unterbrochen oder, wie oben beschrieben, manuell gesteuert werden. Die Unterbrechung der Kühlung kann durch den sich wieder erwärmenden Katheter sehr schmerzhaft für den Patienten werden und weiterhin zu einem Volumenverlust des Katheterschlauches führen, da das Volumen von der Kühlflüssigkeit aufrecht erhalten wird. Unter bestimmten Bedinungen kann der Verlust der Katheterkühlung auch zu einer Patientengefährdung führen. Die manuelle Steuerung hat die oben beschriebenen Nachteile.

Es ist daher wünschenswert, die oben genannten Nachteile zu beheben und eine Kühlmittelpumpe zu Zwecken der Ablation von Herzgewebe anzugeben, welche unabhängig vom Ablationsgenerator steuer- und einsetzbar ist und die Kühlung während aller beschriebener Betriebsmodi sicherstellt, ohne dass der Operateur die Steuerung der Kühlung selbst übernehmen muss. Weiterhin ist es wünschenswert, eine Anordnung zu schaffen, die die Kühlung während einer Ablation sicherstellt und ohne Eingriff oder Steuerung des Operateurs durchgeführt wird.
Die erste Aufgabe wird mit Anspruch 1 gelöst, indem die Steuerung eine Schnittstelle aufweist, die ausgebildet ist, eingangsseitig einen für die Aktivität des Katheters charakteristischen Zustand zu detektieren und ausgangsseitig ein diese Detektion repräsentierendes Anforderungssignal an die Steuerung auszugeben. Durch diese autonome Kühlvorrichtung wird die Arbeit des Operateurs vereinfacht, so dass er sich auf die wesentlichen Verfahrensschritte bei der Ablation konzentrieren kann. Bedingt durch den aktuellen Betriebsmodus des Katheters erkennt die Steuerung der Kühlvorrichtung, welcher Bedarf an Kühlung benötigt wird. Verletzungen des Patienten oder ungewollte Schmerzen durch Fehlbedienung der Pumpe oder falschen (zu niedrigen) Kühlmittelfluss werden dadurch ausgeschlossen.

Vorteilhafterweise sendet die Steuerung der Kühlvorrichtung ein Steuersignal zur Erhöhung der Flussrate an die Kühlmittelpumpe, wenn das Anforderungssignal der Schnittstelle dergestalt ist, dass es die Schwellwertüberschreibung des eingangsseitig detektierten Zustandes repräsentiert. Dieser Schwellenwert betrifft besonders bevorzugt die Temperatur an der Ablationsstelle und liegt dabei vorzugsweise zwischen 37,5 und 40° C. Zusätzlich oder alternativ betrifft der Schwellwert die vom Temperatursensor kommende Spannung, wobei der Schwellenwert je nach technischer Ausführung des Temperatursensors in einem Spannungsbereich zwischen einigen mV (vorzugsweise 10mV) und 5 V liegen kann. Zusätzlich oder alternativ kann der Schwellenwert einen Ablationsfrequenzbereich betreffen, wobei der Schwellenwert in einem Frequenzbereich zwischen 450 und 550 kHz liegt. Ebenso zusätzlich und alternativ zu den genannten Schwellwerten kann der Hochfrequenzstrom auf der Katheterzuleitung detektiert werden, wobei der Schwellenwert zwischen einigen Milliampere und 2 A liegt. Ebenso zusätzlich und alternativ zu den genannten Schwellwerten kann die Impedanz im Behandlungsgebiet detektiert werden, wobei der Schwellenwert zwischen 120 und 250 Ohm liegt.
Dadurch wird der Einsatz der Kühlmittelvorrichtung weiter verbessert und zuverlässiger gemacht, indem die Anforderung für einen Kühlmittelflussänderung anhand von objektiv messbaren und vom Operateur unabhängigen Messwerten durchgeführt wird.

Weiter bevorzugt schickt die Steuerung ein Steuersignal an die Kühlmittelpumpe, das vom Anforderungssignal der Schnittstelle abhängig ist, so dass die Kühlmittelpumpe eine dem eingangsseitig detektierten Zustand entsprechende Flussrate erzeugt. Dadurch wird das System noch mehr verbessert, indem eine Flussrate erzeugt wird, die durch ihren direkten Bezug auf den Zustand am Ablationskatheter bedingt ist. Mit anderen Worten stellt die Steuerung die Pumpe so ein, dass ein kontinuierlicher und stufenlos einstellbarer Kühlmittelfluss entsteht, der eine direkte Funktion des detektierten Zustandes (Temperatur, Strom, Frequenz und/oder Impedanz) ist. So kann die Kühlvorrichtung auf kleinste Änderungen der Temperatur, des Stroms, der Energie und/oder der Impedanz adäquat und angepasst reagieren. Dies führt zu einer zuverlässigen, sicheren Therapie bei möglichst gleichbleibenden Bedingungen.

Vorzugsweise steuert die Steuerung die Kühlmittelpumpe so an, dass diese ohne Anforderungssignal der Schnittstelle die niedere Flussrate erzeugt. Mit anderen Worten wird ein kontinuierlicher Kühlmittelfluss erreicht, der die Nachkühlung nach erfolgter Ablation sicherstellt und weiter zur Verminderung von Schmerzen oder Verletzungen beiträgt. Er verhindert auch, dass Blut in die Kühlmittelbahn eindringt und sie verstopft. Außerdem wird der Operateur so in der Lage versetzt, den Ablationsgenerator zu wechseln, ohne dass die Pumpe ausgetauscht werden muss. Es wird also eine immerwährende Kühlung sichergestellt.

Im einfachsten Fall handelt es sich bei der Schnittstelle um ein Kabel, welches an seinem von der Steuerung abgewandten Ende einen Adapter zur galvanisch direkten oder indirekten Detektion aufweist. Dieser Adapter kann verschiedene Formen annehmen. Beispielsweise kann der Adapter ösen-, röhren oder zangenförmig ausgebildet sein. Die Form birgt den Vorteil, dass der Adapter universell an allen gängigen Kathetern oder deren Zuleitungen leicht befestigt werden kann. Dazu wird die Öse oder das Rohr über den Katheterschaft geschoben, so dass beispielsweise ein elektrisches Feld gemessen werden kann, wenn eine Ablation stattfindet. Gemäß einer Alternative ist der Adapter als Stecker ausgestaltet, so dass dieser beispielsweise direkt am Generator oder am Katheter angeschlossen werden kann. Am Katheter ist dies beispielsweise an der Anschlussleitung für die Temperaturmessung möglich, an der die Temperatursensoren am distalen Ende des Ablationskatheters elektrisch verbunden sind. Der Kühlmittelfluss kann dabei also in Abhängigkeit der gemessenen Temperatur eingestellt werden. Weiterhin ist es möglich, ausgehend von der gemessenen Impedanz auf die Temperatur an der Behandlungsstelle zu schließen, da die Impedanz steigt, wenn die Temperatur über der physiologisch normalen Temperatur ist.

In einer Ausführung weist das genannte Kabel eine elektrische Schaltung auf, die den eingangsseitig detektierten, für die Aktivität des Katheters charakteristischen Zustand in ein ausgangsseitiges Anforderungssignal umwandelt. Dies kann beispielsweise ein A-/D-Wandler sein. Besonders bevorzugt umfasst die Schaltung weiterhin ein Mittel, das den ausreichenden Sitz des Adapters zur fehlerfreien Detektion des für die Aktivität des Katheters charakteristischen Zustandes sicherstellt, vorzugsweise umfasst dieses Mittel einen Hall-Sensor am Adapter. Weiterhin kann das Mittel der Schaltung einen akustischen oder visuellen Signalgeber umfassen, der besonders bevorzugt eine Leuchtdiode ist. Damit wird anzeigt, ob der Adapter ausreichend am Katheter sitzt und ein fehlerfreie Detektion möglich ist. Dies bewirkt eine sichere Messung, um noch weiter auszuschließen, dass der Patient während der Behandlung geschädigt wird.

In einer weiteren Ausführung weist die Schnittstelle an seinem von der Steuerung abgewandten Ende einen Adapter zur berührungslosen Nah- oder Fernfelddetektion auf, welcher bevorzugt als Hochfrequenzantenne ausgelegt ist. Dies erhöht weiter die Bedieungsfreundlichkeit für den Operateur, da das störende Kabel entfallen kann.

Die zweite Aufgabe wird mit den Merkmalen des Anspruchs 10 gelöst, indem eine Anordnung zur Ablation von Gewebe in einem menschlichen oder tierischen Körper dargestellt ist. Diese Anordnung umfasst neben der soeben beschriebenen Kühlvorrichtung einen lang gestreckten Ablationskatheter mit einem Anschlussstück und einem Katheterschlauch, der über ein Gefäß an die Behandlungsstelle geführt wird. Weiterhin umfasst die Anordnung einen mit dem Katheter elektrisch verbundener Ablationsgenerator, der ausgangsseitig einen hochfrequenten Energieimpuls oder ein hochfrequentes Energiefeld erzeugt und über eine Anschlussleitung an den Ablationskatheter weiterleitet.
Die Kühlmittelpumpe der Kühlvorrichtung ist ausgebildet, ein flüssiges Kühlmedium über eine Anschlussleitung in mindestens eine Kühlbahn des Ablationskatheters zu pumpen. Die Erfindung zeichnet sich dadurch aus, dass die Schnittstelle der Kühlvorrichtung eingangsseitig mit dem Generator und/oder dem Katheter galvanisch direkt und/oder galvanisch indirekt und/oder über Nah-/Fernfelddetektion verbunden ist, um den für die Aktivität des Katheters charakteristischen Zustand zu detektieren. Der Operateur wird damit frei in seiner Geräte- und Katheterwahl und hat somit mehrere Möglichkeiten die Ablation optimiert durchzuführen, da er unabhängig vom Katheter den Generator austauschen kann, während die Kühlung weiterhin sichergestellt ist. Diese Anordnung ist dabei so modular, dass die universell eingesetzte Pumpe mit jeder Art von Generator und Katheter eingesetzt werden kann.

Bevorzugter Weise weist die Schnittstelle der Kühlvorrichtung einen Adapter auf, der mit dem Generator, der elektrischen Anschlussleitung des Generators oder dem Anschlussstück des Ablationskatheters verbunden ist.

Die Erfindung wird anhand zweier Ausführungsbeispiele beschrieben. Dabei zeigt
- Fig 1: ein erstes Ausführungsbeispiel durch Abnahme eines Hochfrequenzsignals an der elektrischen Anschlussleitung, und
- Fig 2: ein zweites Ausführungsbeispiel durch Messen einer Sensorspannung eines Temperatursensors an der Katheterspitze.

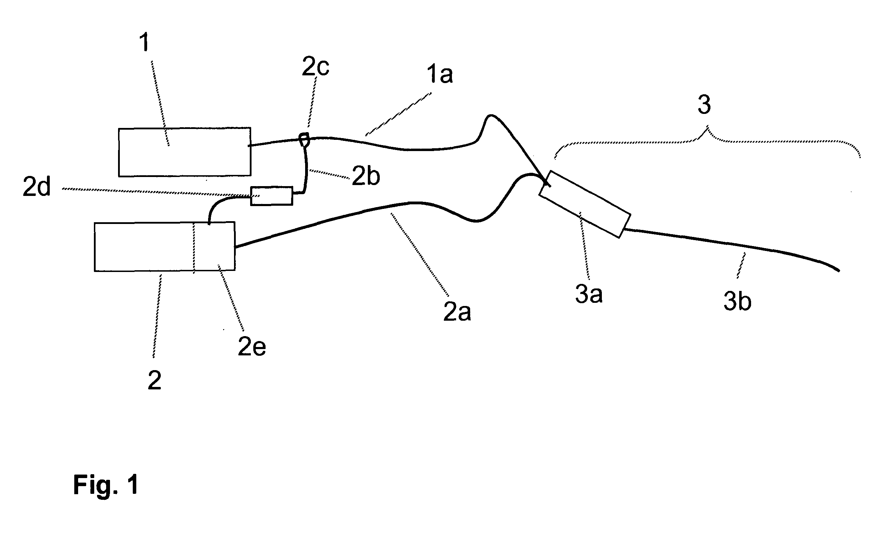

Fig. 1 zeigt eine Ablationsanordnung, bestehend aus einem Ablationsgenerator 1, einer Kühlmittelpumpe 2 und einem Katheter 3, hier dargestellt mit einem am proximalen Ende befindlichen Anschlussstück 3a, mit Hilfe dessen der Generator 1 und die Pumpe 2 angeschlossen werden können. Das Anschlussstück 3a stellt dabei die elektrische Verbindung zwischen dem Generator 1 und den nicht dargestellten elektrischen Zuleitungen und eine dichte Verbindung zwischen Kühlmittelpumpe 2 und dem nicht dargestellten Kühlmittelkanal her. Der lang gestreckte Katheterschlauch ist andeutungsweise mit 3b bezeichnet. Ebenfalls nicht dargestellt sind die Elektrodenpole zur Übertragung der hochfrequenten Energie an das Gewebe und die Steuereinrichtung.

Der Generator ist mit einer elektrischen Anschlussleitung 1a verbunden, während die Pumpe mit einer hohlen, Kühlmittel führenden Anschlussleitung 2a mit dem Anschlussstück 3a verbunden ist. Als Kühlmittel wird physiologische Kochsalzlösung verwendet.

Weiter umfasst die Pumpe 2 eine Steuerleitung 2b, welche einerseits elektrisch mit der elektrischen Anschlussleitung 1a verbunden ist. Die Steuerleitung kann erfindungsgemäß auch an das Anschlussstück 3a oder an die elektrische Zuleitung des Katheters 3 angeschlossen werden. Die Steuerleitung 2b dient zur Detektion eines hochfrequenten Impulses oder eines hochfrequenten Feldes, das vom Generator 1 über die Anschlussleitung 1a an den Katheter 3 abgegeben wird. Die elektrische Verbindung zwischen der Steuerleitung 2b und der Anschlussleitung 1a ist erfindungsgemäß mit einem Adapter 2c - geeignet sind handelsübliche und/oder normierte Stecker, elektrische Klemmadapter wie eine Klemmzange oder andere geeignete Mittel - hergestellt. Möglich sind aber ebenso Ösen oder Rohrabschnitte, die leicht über den Katheter geschoben werden.

Andererseits ist die Steuerleitung 2b eingangsseitig an eine weitere elektrische Steuerung 2e angeschlossen, welche die Pumpe steuert.

Die Steuerleitung 2b oder der Adapter 2c enthält eine elektronische Schaltungseinheit 2d, die einen an der Anschlussleitung 1a anliegenden hochfrequenten elektrische Impuls oder ein Feld erfasst.
Dabei misst die elektrische Schaltungseinheit 2d die Feldstärke, die an der Anschlussleitung 1a anliegt. Übersteigt oder unterschreitet die gemessene Feldstärke in einem definierten Frequenzband einen Schwellwert, schaltet die Elektronik des Zangenadapters einen Triggerpegel um. Dieser Triggerpegel wird verwendet, um die Steuerung 2e der Pumpe 2 anzusteuern, welche zwischen zwei Flussraten umschaltet. Liegt also an der Anschlussleitung 1a und damit am Adapter 2c eine hohe Feldstärke an, so gestaltet sich das Ausgangssignal der Schaltungseinheit 2d so, dass die Steuerung 2e auf die höhere Flussrate umschaltet. Dies ist der Fall, wenn ein Ablationsvorgang gestartet wird. Wird der Ablationsvorgang beendet, unterschreitet die Feldstärke den Schwellenwert und das ausgangsseitige Signal der Schaltungseinheit 2d ist dergestalt, dass die Steuerung 2e die Pumpe auf eine niedere Flussrate umschaltet.
Vorteilhaft kann die Schaltungseinheit 2d ein Verzögerungsglied enthalten, welches nur beim Unterschreitungsvorgang ein verzögertes Ausgangssignal an die Steuerung 2e weiterleitet, um die vollständige Kühlung zu erreichen.

Somit ist sichergestellt, dass die Kühlmittelpumpe unabhängig eine minimale dauerhafte Flussrate ermöglicht, auch wenn der Generator 1 ausgeschaltet ist oder ausgewechselt wird. Die Kühlung und die Funktion des Katheters sind also weiterhin möglich.

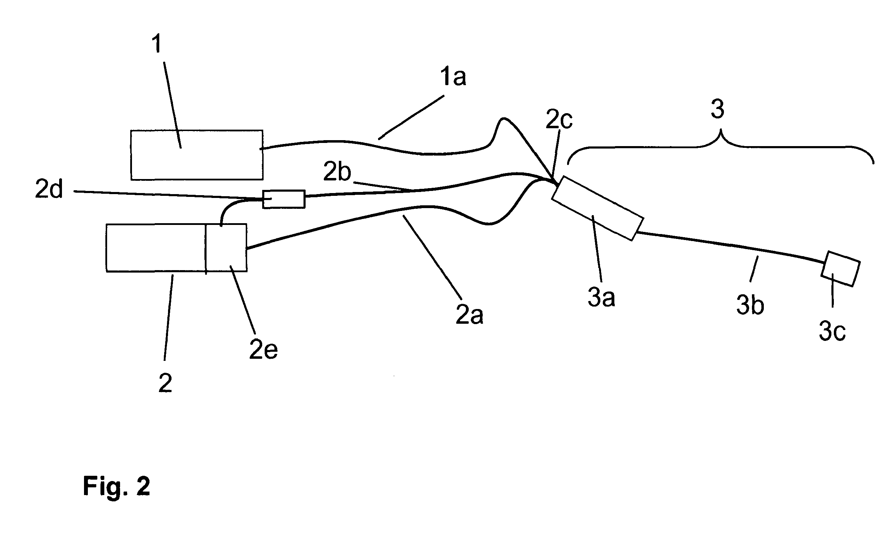

Fig. 2 zeigt eine Ablationsanordnung, bestehend aus einem Ablationsgenerator 1, einer Kühlmittelpumpe 2 und einem Katheter 3, hier dargestellt mit einem am proximalen Ende befindlichen Anschlussstück 3a, mit Hilfe dessen der Generator 1 und die Pumpe 2 angeschlossen werden können. Das Anschlussstück 3a stellt dabei die elektrische Verbindung zwischen dem Generator 1 und den nicht dargestellten elektrischen Zuleitungen und eine dichte Verbindung zwischen Kühlmittelpumpe 2 und dem nicht dargestellten Kühlmittelkanal her. Der lang gestreckte Katheterschlauch ist andeutungsweise mit 3b bezeichnet. Ebenfalls nicht dargestellt sind die Elektrodenpole zur Übertragung der hochfrequenten Energie an das Gewebe und die Steuereinrichtung.

Der Generator ist mit einer elektrischen Anschlussleitung 1a verbunden, während die Pumpe mit einer hohlen, Kühlmittel führenden Anschlussleitung 2a mit dem Anschlussstück 3a verbunden ist. Als Kühlmittel wird physiologische Kochsalzlösung verwendet.

Weiter umfasst die Pumpe 2 eine Steuerleitung 2b, welche an das Anschlussstück 3a oder an die elektrische Zuleitung des Katheters 3 angeschlossen ist. Die Steuerleitung 2b dient zur Detektion des Signals, das vom Temperatursensor 3c an der Katheterspitze gemessen und durch den Katheter 3 und die elektrische Anschlussleitung 1a an den Generator 1 abgegeben wird. Die elektrische Verbindung zwischen der Steuerleitung 2b und der Anschlussleitung 1a ist erfindungsgemäß mit einem Adapter 2c - geeignet sind handelsübliche und/oder normierte Stecker- hergestellt.

Andererseits ist die Steuerleitung 2b eingangsseitig an eine weitere elektrische Steuerung 2e angeschlossen, welche die Pumpe steuert.

Die Steuerleitung 2b oder der Adapter 2c enthält eine elektronische Schaltungseinheit 2d, die ein am Temperatursensor 3c gemessenes und durch den Katheter 3 und die Anschlussleitung 1a an den Generator 1 abgegebenes Temperatursignal erfasst.
Dabei misst die elektrische Schaltungseinheit 2d die Spannung des Temperatursignals, die an der Anschlussleitung 1a anliegt. Übersteigt oder unterschreitet die gemessene Spannung einen definierten Spannungsschwellwert, schaltet die elektronische Schaltungseinheit 2d einen Triggerpegel um. Dieser Triggerpegel wird verwendet, um die Steuerung 2e der Pumpe 2 anzusteuern, welche zwischen zwei Flussraten umschaltet. Liegt also an der Anschlussleitung 1a und damit am Adapter 2c eine hohe Spannung an, so gestaltet sich das Ausgangssignal der Schaltungseinheit 2d so, dass die Steuerung 2e auf die höhere Flussrate umschaltet. Dies ist der Fall, wenn ein Ablationsvorgang gestartet wird und sich dadurch die Temperatur in der Katheterspitze erhöht. Wird der Ablationsvorgang beendet, unterschreitet die vom Temperatursensor kommende Spannung den Schwellenwert und das ausgangsseitige Signal der Schaltungseinheit 2d ist dergestalt, dass die Steuerung 2e die Pumpe auf eine niedere Flussrate umschaltet.

Somit ist sichergestellt, dass die Kühlmittelpumpe 2 unabhängig eine minimale dauerhafte Flussrate ermöglicht, auch wenn der Generator 1 ausgeschaltet ist oder ausgewechselt wird. Die Kühlung und die Funktion des Katheters 3 sind also weiterhin möglich.

## Patentansprüche

1. Vorrichtung zur Kühlung von Ablationskathetern, umfassend
- eine Kühlmittelpumpe (2), welche auf Anforderung flüssiges Kühlmedium mit mindestens einer niederen und einer erhöhten Flussrate in einer Anschlussleitung (2a) bereitstellt, und
eine Steuerung (2e), die die Kühlmittelpumpe so steuert, dass diese die angeforderte mindestens niedere oder erhöhte Flussrate erzeugt, wobei die Steuerung eine Schnittstelle (2b) aufweist, die ausgebildet ist, eingangsseitig einen für die Aktivität des Katheters charakteristischen Zustand zu detektieren und ausgangsseitig ein diese Detektion repräsentierendes Anforderungssignal an die Steuerung auszugeben
**dadurch gekennzeichnet, dass**
die Schnittstelle (2b) ein Kabel ist, welches an seinem von der Steuerung (2e) abgewandten Ende einen zangenförmig ausgestalteten Adapter (2c) zur galvanisch direkten oder indirekten Detektion aufweist.

2. Vorrichtung nach Anspruch 1, **dadurch gekennzeichnet, dass** die Steuerung (2e) ein Steuersignal zur Erhöhung der Flussrate an die Kühlmittelpumpe (2) sendet, wenn das Anforderungssignal der Schnittstelle (2b) dergestalt ist, dass es die Schwellwertüberschreibung des eingangsseitig detektierten Zustandes repräsentiert, wobei der Schwellenwert vorzugsweise in einem Temperaturbereich zwischen 37,5 und 40° C und/oder in einem Frequenzbereich zwischen 450 und 550 kHz und/oder in einen Bereich des Hochfrequenzstromes zwischen wenigen mA und 2 A und/oder einem Spannungsbereich zwischen wenigen mV und 5 V und/oder einem Impedanzbereich zwischen 120 und 250 Ohm liegt.

3. Vorrichtung nach Anspruch 1, **dadurch gekennzeichnet, dass** die Steuerung (2e) ein Steuersignal an die Kühlmittelpumpe (2) sendet, das vom Anforderungssignal der Schnittstelle (2b) abhängig ist, so dass die Kühlmittelpumpe eine dem eingangsseitig detektierten Zustand entsprechende Flussrate erzeugt.

4. Vorrichtung nach einem der Ansprüche 1 bis 3, **dadurch gekennzeichnet, dass** die die Steuerung (2e) die Kühlmittelpumpe (2) so steuert, dass diese ohne Anforderungssignal der Schnittstelle (2e) die niedere Flussrate erzeugt.

5. Vorrichtung nach Anspruch 1, **dadurch gekennzeichnet, dass** das Kabel eine elektrische Schaltung (2d) aufweist, die den eingangsseitig detektierten, für die Aktivität des Katheters charakteristischen Zustand in ein ausgangsseitiges Anforderungssignal umwandelt.

6. Vorrichtung nach Anspruch 5, **dadurch gekennzeichnet, dass** die Schaltung (2d) weiterhin ein Mittel umfasst, das den festen Sitz des Adapters (2c) zur fehlerfreien Detektion des für die Aktivität des Katheters charakteristischen Zustandes sicherstellen, vorzugsweise umfasst dieses Mittel einen Hall-Sensor am Adapter (2c).

7. Vorrichtung nach Anspruch 6, **dadurch gekennzeichnet, dass** das Mittel der Schaltung (2d) weiterhin einen akustischen oder visuellen Signalgeber, besonders bevorzugt eine Leuchtdiode, umfasst, welche anzeigen, dass der Adapter fest sitzt und ein fehlerfreie Detektion möglich ist.

8. Vorrichtung nach einem der Ansprüche 1 bis 4, **dadurch gekennzeichnet, dass** die Schnittstelle (2b) an seinem von der Steuerung abgewandten Ende einen Adapter (2c) zur berührungslosen Nah- oder Fernfelddetektion aufweist, welcher bevorzugt als Hochfrequenzantenne ausgelegt ist.

9. Anordnung zur Ablation von Gewebe in einem menschlichen oder tierischen Körper, umfassend
- einen lang gestreckten Ablationskatheter (3) mit einem Anschlussstück (3a) und einem Katheterschlauch (3b), welcher über ein Gefäß an die Behandlungsstelle geführt wird
- einen mit dem Katheter elektrisch verbundenen Ablationsgenerator (1), welcher ausgangsseitig einen hochfrequenten Energieimpuls oder ein hochfrequentes Energiefeld erzeugt und über eine Anschlussleitung (1a) an den Ablationskatheter weiterleitet,
- eine mit dem Katheter verbundene Kühlmittelpumpe (2) gemäß einem oder mehrerer der Ansprüche 1 bis 8, welche ein flüssiges Kühlmedium über eine Anschlussleitung (2a) in mindestens eine Kühlbahn des Ablationskatheters pumpt,
**dadurch gekennzeichnet, dass**
die Schnittstelle (2b) eingangsseitig mit dem Generator (1) und/oder dem Katheter (3) galvanisch direkt und/oder galvanisch indirekt und/oder über Nah/Fernfelddetektion verbunden ist, um den für die Aktivität des Katheters charakteristischen Zustand zu detektieren.

10. Anordnung nach Anspruch 9, **dadurch gekennzeichnet, dass** die Schnittstelle (2b) einen Adapter (2c) aufweist, der mit dem Generator (1), der elektrischen Anschlussleitung (1a) des Generators (1) oder dem Anschlussstück (3a) des Ablationskatheters (3) verbunden ist.

## Claims

1. A device for cooling ablation catheters, comprising
- a coolant pump (2), which when prompted supplies a liquid cooling medium at at least one lower and a higher flow rate in a connecting line (2a), and
a control unit (2e), which controls the coolant pump so that the same generates the requested at least lower or higher flow rate, wherein the control unit comprises an interface (2b), which is designed, on the input side, to detect a state that is characteristic of the activity of the catheter and, on the output side, to output a request signal representing this detection to the control unit,
**characterized in that**
the interface (2b) is a cable, which at the end thereof facing away from the control unit (2e) comprises a clamp-shaped adapter (2c) for the galvanically direct or indirect detection.

2. The device according to claim 1, **characterized in that** the control unit (2e) sends a control signal for increasing the flow rate to the coolant pump (2) if the request signal from the interface (2b) is such that the signal represents the crossing above the threshold value of the state detected on the input side, wherein the threshold value is preferably in a temperature range between 37.5 and 40°C and/or in a frequency range between 450 and 550 kHz and/or in a range of the radio frequency current between a few mA and 2 A and/or in a voltage range between a few mV and 5 V and/or in an impedance range between 120 and 250 ohm.

3. The device according to claim 1, **characterized in that** the control unit (2e) sends a control signal to the coolant pump (2) which is dependent on the request signal of the interface (2b), so that the coolant pump generates a flow rate that corresponds to the state detected on the input side.

4. A device according to any one of claims 1 to 3, **characterized in that** the control unit (2e) controls the coolant pump (2) so that the same generates the lower flow rate without the request signal from the interface (2e).

5. The device according to claim 1, **characterized in that** the cable comprises an electric circuit (2d), which converts the state that is characteristic of the activity of the catheter, as detected at the input side, into an output-side request signal.

6. The device according to claim 5, **characterized in that** the circuit (2d) further comprises a means that assures firm seating of the adapter (2c) for the fault-free detection of the state that is characteristic of the activity of the catheter, this means preferably comprising a Hall sensor at the adapter (2c)

7. The device according to claim 6, **characterized in that** the means of the circuit (2d) further comprises an acoustic or visual signal generator, particularly preferably a light-emitting diode, which indicates that the adapter is firmly seated and fault-free detection is possible.

8. A device according to any one of claims 1 to 4, **characterized in that** the end of the interface (2b) facing away from the control unit comprises an adapter (2c) for the non-contact near or far field detection, which is preferably designed as a radio frequency antenna.

9. An array for ablating tissue in a human or animal body, comprising
- an elongate ablation catheter (3) comprising a connection piece (3a) and a catheter tube (3b), which is guided via a vessel to the treatment site,
- an ablation generator (1), which is electrically connected to the catheter and which, on the output side, generates a radio frequency energy pulse or a radio frequency energy field and forwards the same to the ablation catheter by way of a connecting cable (1a), and
- a coolant pump (2) according to one or more of claims 1 to 8, which is connected to the catheter and pumps a liquid cooling medium via a connecting line (2a) to at least one cooling path of the ablation catheter,
**characterized in that**
at the input side, the interface (2b) is connected galvanically directly and/or galvanically indirectly and/or by way of near/far field detection to the generator (1) and/or the catheter (3), so as to detect the state that is characteristic of the activity of the catheter.

10. The array according to claim 9, **characterized in that** the interface (2b) comprises an adapter (2c), which is connected to the generator (1), the electrical connecting cable (1a) of the generator, or the connection piece (3a) of the ablation catheter.

## Revendications

1. Dispositif pour le refroidissement de cathéters d'ablation, comprenant
- une pompe de liquide de refroidissement (2) mettant à disposition un liquide de refroidissement sur demande dans une conduite de raccordement (2a), avec au moins un débit bas et un débit élevé, et
une commande (2e) commandant la pompe à liquide de refroidissement de manière à ce que celle-ci produise le débit bas ou élevé demandé, la commande comportant une interface (2b) conçue pour détecter côté entrée un état caractéristique de l'activité du cathéter, et pour envoyer côté sortie un signal de requête représentant cette détection, à la commande,
**caractérisé en ce que**
l'interface (2b) est un câble comportant, à son extrémité détournée de la commande (2e), un adaptateur (2c) conçu en forme de pince, pour une détection galvaniquement directe ou indirecte.

2. Dispositif selon la revendication 1, **caractérisé en ce que** la commande (2e) envoie un signal de commande à la pompe de liquide de refroidissement (2) pour augmenter le débit, lorsque le signal de requête de l'interface (2b) est conçu de manière à représenter le dépassement de valeur seuil de l'état détecté côté entrée, dans lequel la valeur seuil est de préférence comprise dans une plage de température entre 37,5 et 40°C et/ou dans une plage de fréquence entre 450 et 550 kHz et/ou dans une plage de courant à haute fréquence entre quelques mA et 2A et/ou dans une plage de tension entre quelques mV et 5V et/ou dans une plage d'impédance entre 120 et 250 Ohm.

3. Dispositif selon la revendication 1, **caractérisé en ce que** la commande (2e) envoie à la pompe de liquide de refroidissement (2) un signal de commande dépendant du signal de requête de l'interface (2b), de manière à ce que la pompe de liquide de refroidissement produise le débit correspondant à l'état détecté côté entrée.

4. Dispositif selon l'une des revendications 1 à 3, **caractérisé en ce que** la commande (2e) commande la pompe de liquide de refroidissement (2) de manière à ce que celle-ci produise le débit bas sans le signal de requête de l'interface (2e).

5. Dispositif selon la revendication 1, **caractérisé en ce que** le câble comporte un circuit électrique (2d) transformant l'état caractéristique de l'activité du cathéter détecté côté entrée en un signal de requête côté sortie.

6. Dispositif selon la revendication 5, **caractérisé en ce que** le circuit (2d) comprend en outre un moyen permettant de sécuriser l'ajustement fixe de l'adaptateur (2c), pour une détection correcte de l'état caractéristique de l'activité du cathéter, ce moyen comprenant de préférence un capteur Hall sur l'adaptateur (2c).

7. Dispositif selon la revendication 6, **caractérisé en ce que** le moyen du circuit (2d) comprend en outre un indicateur de signal visuel ou sonore, de manière particulièrement préférée une diode lumineuse, indiquant que l'adaptateur est monté fixement et que la détection peut se dérouler correctement.

8. Dispositif selon l'une des revendications 1 à 4, **caractérisé en ce qu'**à son extrémité détournée de la commande, l'interface (2b) comporte un adaptateur (2c) pour la détection d'un champ lointain ou proche sans contact, conçu de préférence comme une antenne haute fréquence.

9. Agencement pour l'ablation de tissu dans un corps humain ou d'animal, comprenant
- un cathéter d'ablation (3) allongé avec une pièce de raccordement (3a) et un tube de cathéter (3b) guidé vers le point de traitement par le biais d'un vaisseau,
- un générateur d'ablation (1) électriquement relié au cathéter, produisant côté sortie une impulsion d'énergie à haute fréquence ou un champ énergétique à haute fréquence et guidant celle-ci/celui-ci vers le cathéter d'ablation par une ligne de raccordement (1a),
- une pompe de liquide de refroidissement (2) reliée au cathéter, selon l'une ou plusieurs des revendications 1 à 8, pompant un liquide de refroidissement vers au moins une voie de refroidissement du cathéter d'ablation, par le biais d'une conduite de raccordement (2a),
**caractérisé en ce que**
côté entrée, l'interface (2b) est reliée au générateur (1) et/ou au cathéter (3), de façon galvaniquement directe et/ou galvaniquement indirecte et/ou par une détection de champ proche/lointain, pour détecter l'état caractéristique de l'activité du cathéter.

10. Agencement selon la revendication 9, **caractérisé en ce que** l'interface (2b) comporte un adaptateur (2c) relié au générateur (1), à la ligne de raccordement électrique (1a) du générateur (1) ou avec la pièce de raccordement (3a) du cathéter d'ablation (3).
